(19) Europäisches Patentamt
European Patent Office
Office européen des brevets

(11) Numéro de publication : **0 488 897 B1**

(12) # FASCICULE DE BREVET EUROPEEN

(45) Date de publication du fascicule du brevet : **02.02.94 Bulletin 94/05**

(21) Numéro de dépôt : **91403230.5**

(22) Date de dépôt : **29.11.91**

(51) Int. Cl.[5] : **C07C 281/18,** C07C 281/16, C07D 233/48, C07D 233/50, C07D 233/52, A61K 31/155, A61K 31/415

(54) **Nouveaux dérivés de guanidine, leur procédé de préparation et les compositions pharmaceutiques qui les contiennent.**

Le dossier contient des informations techniques présentées postérieurement au dépôt de la demande et ne figurant pas dans le présent fascicule.

(30) Priorité : **29.11.90 FR 9014898**

(43) Date de publication de la demande : **03.06.92 Bulletin 92/23**

(45) Mention de la délivrance du brevet : **02.02.94 Bulletin 94/05**

(84) Etats contractants désignés : **AT BE CH DE DK ES FR GB GR IT LI LU NL SE**

(56) Documents cités :
**DE-B- 1 205 521**
**FR-A- 2 212 143**
**FR-A- 2 444 673**
**GB-A- 1 223 491**
**US-A- 4 136 188**

(73) Titulaire : **ADIR ET COMPAGNIE**
**1 rue Carle Hébert**
**F-92415 Courbevoie Cédex (FR)**

(72) Inventeur : **Malen, Charles**
**3, allée Traverière**
**F-94260 Fresnes (FR)**
Inventeur : **Lacoste, Jean-Michel**
**103, rue Brancas**
**F-92310 Sevres (FR)**
Inventeur : **De Nanteuil, Guillaume**
**12 rue du Chemin Vert**
**F-92150 Suresnes (FR)**

EP 0 488 897 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés de guanidine, leur procédé de préparation et les compositions pharmaceutiques qui les contiennent.

De nombreux dérivés de guanidines acycliques, de 2-aminoimidazolines mieux définies comme guanidines cycliques ainsi que d'imidazolines ont été décrites dans la littérature. La plupart de ces molécules présentent des affinités pour les récepteurs $\alpha_2$-adrénergiques.

Or il a été récemment mis en évidence qu'il existait des récepteurs spécifiques "imidazoline-guanidine" différents des récepteurs $\alpha_2$-adrénergiques tant au niveau du système nerveux central qu'au niveau périphérique (P. Bousquet et Coll. Eur. J. Pharmacol., 150(3), 401, 1988 et A. PARINI et Coll, J. Biol. Chem., 264(20), 11874, 1989).

Les composés de la présente invention, outre le fait qu'ils soient nouveaux, présentent une affinité tout à fait spécifique pour ces récepteurs "imidazoline-guanidine" que l'on peut également nommer récepteurs "endazoliniques". Cette affinité est d'autant plus intéressante qu'elle est accompagnée d'une très grande sélectivité non seulement vis-à-vis des récepteurs $\alpha_1$ et $\alpha_2$ adrénergiques mais encore vis-à-vis des récepteurs 5-HT.

La présence des récepteurs "endazoliniques" au niveau du système nerveux central, au niveau hépatique, au niveau du tissu adipeux ainsi qu'au niveau pulmonaire, rend donc les composés de l'invention utilisables, en tant qu'agoniste ou antagoniste vis-à-vis de ces récepteurs, aussi bien dans le traitement de la dépression, de l'hypertension artérielle, des troubles vasculaires que dans le traitement du diabète, de l'obésité et des maladies pulmonaires.

L'état antérieur de la technique est illustré notamment dans les brevets GB-A-1223491, DE-B-1205521, US-A-4136188, FR-A-2444673 et FR-A-2212143.

L'invention concerne plus spécifiquement de nouveaux dérivés de guanidine répondant à la formule générale (I) :

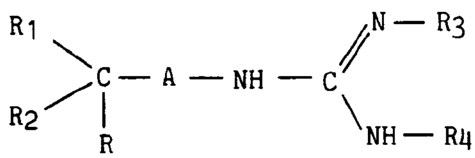

(I)

dans laquelle :

$R_1$ représente - un groupement phényle éventuellement substitué par un ou plusieurs atomes d'halogène ou groupements alkyle ($C_1$-$C_6$) linéaire ou ramifié, hydroxy, alkoxy ($C_1$-$C_6$) linéaire ou ramifié,
- un groupement cycloalkyle contenant de 3 à 7 atomes de carbone,

$R_2$ représente un groupement trifluorométhyle ou un groupement cycloalkyle contenant de 3 à 4 atomes de carbone,

A représente un groupement $-CH_2-$, $-CH = N-$, $= N-$,

$R_3$ et $R_4$ identiques ou différents représentent un atome d'hydrogène, un groupement alkyle ($C_1$-$C_6$) linéaire ou ramifié, ou forment avec les atomes d'azote auxquels ils sont attachés un hétérocycle à 5 ou 6 chaînons,

R représente soit un atome d'hydrogène dans le cas où A représente un groupement $-CH_2-$ ou $-CH = N-$, soit une liaison du groupement A quand celui-ci est représenté par $= N-$,
à la condition toutefois que, lorsque A représente un groupement $=N-$ et $R_1$ un groupement phényle éventuellement substitué, $R_2$ ne représente pas simultanément un groupement cycloalkyle ($C_3$-$C_4$),

leurs énantiomères et épimères ainsi que leurs sels d'addition à un acide pharmaceutiquement acceptable.

Parmi les acides pharmaceutiquement acceptables, on peut citer, à titre non limitatif, les acides chlorhydrique, sulfurique, tartrique, maléique, fumarique, méthane sulfonique, camphorique, etc...

L'invention s'étend aussi au procédé de préparation des composés de formule (I) caractérisé en ce que l'on utilise comme produit de départ une cétone de formule (II) :

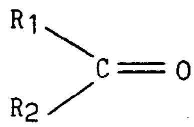

(II)

dans laquelle $R_1$ et $R_2$ ont la même signification que dans la formule (I) que l'on soumet

1/ soit

à l'action d'un composé de formule (III) dans un solvant organique

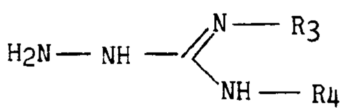

(III)

dans laquelle $R_3$ et $R_4$ ont la même signification que dans la formule (I)
pour conduire à un composé de formule (I/a), cas particulier des composés de formule (I)

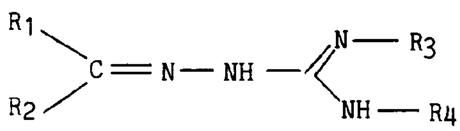

(I/a)

dans laquelle $R_1$, $R_2$, $R_3$ et $R_4$ ont la même signification que dans la formule (I), A représente un groupement = N- et R une liaison de ce groupement,

2/ soit

à l'action du chlorure de méthoxyméthyltriphénylphosphonium en présence d'une base forte pour conduire à l'éther d'énol de formule (IV)

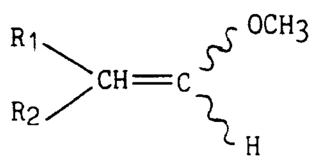

(IV)

dans laquelle $R_1$ et $R_2$ ont la même signification que dans la formule (I)
que l'on hydrolyse en milieu acide pour conduire à l'aldéhyde de formule (V)

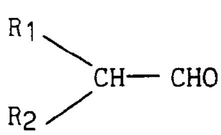

(V)

dans laquelle $R_1$ et $R_2$ ont la même signification que dans la formule (I),
que l'on soumet
ou bien
à l'action d'un composé de formule (III)

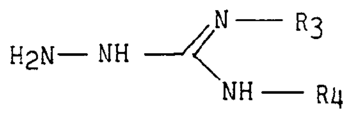

(III)

dans laquelle $R_3$ et $R_4$ ont la même signification que dans la formule (I) pour conduire à un composé de formule (I/c), cas particulier des composés de formule (I)

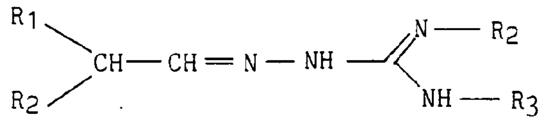

(I/c)

dans laquelle $R_1$, $R_2$, $R_3$, $R_4$ ont la même signification que dans la formule (I), A représente un groupement -CH =N- et R un atome d'hydrogène,

ou bien

à l'action d'un composé de formule (VI) en présence de pyridine

$H_2N$ - O - R' (VI)

dans laquelle R' représente un atome d'hydrogène ou un groupement alkyle ($C_1$-$C_6$), pour conduire à un composé de formule (VII)

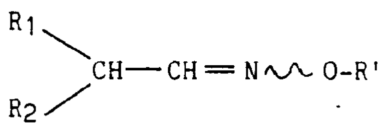

(VII)

dans laquelle $R_1$, $R_2$ et R' ont la même signification que précédemment que l'on réduit par hydrogénation catalytique ou en présence d'un hydrure mixte de métal alcalin pour conduire à une amine de formule (VIII)

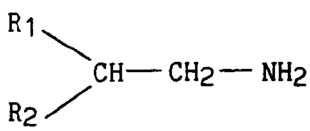

(VIII)

dans laquelle $R_1$ et $R_2$ ont même signification que dans la formule (I), que l'on soumet à l'action d'un composé de formule (IX)

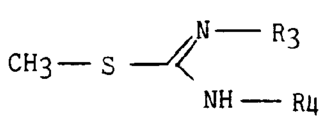

(IX)

dans laquelle $R_3$ et $R_4$ ont la même signification que dans la formule (I) pour conduire à un composé de formule (I/d), cas particulier des composés de formule (I)

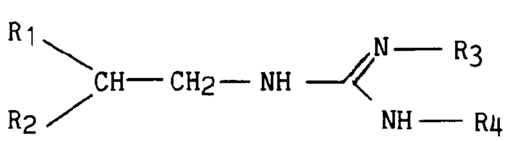

(I/d)

dans laquelle $R_1$, $R_2$, $R_3$, $R_4$ ont la même signification que dans la formule (I), A représente un groupement -$CH_2$- et R un atome d'hydrogène,
composés de formule (I/a), (I/c) et (I/d) qui constituent l'ensemble des composés de formule (I), que l'on purifie, le cas échéant, par des techniques classiques de purification, dont on sépare, si on le souhaite, les isomères par des techniques classiques de séparation et que l'on transforme, si nécessaire, en leurs sels d'addition à un acide pharmaceutiquement acceptable.

La présente invention a également pour objet les compositions pharmaceutiques renfermant comme principe actif au moins un composé de formule générale (I) ou un de ses sels d'addition à un acide pharmaceutiquement acceptable, seul ou en combinaison avec un ou plusieurs excipients ou véhicules inertes, non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les sachets, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention et ne la limitent en aucune façon.

**EXEMPLE 1 : (2,2-Dicyclopropyléthylidényl)-hydrazino-2-imidazoline**

STADE A : Dicyclopropylacétaldéhyde

A une suspension de 1,2 moles d'hydrure de sodium dans 1,4 litre de diméthylsulfoxyde est ajoutée rapidement, sous atmosphère d'azote, 0,77 mole de dicyclopropylcétone.

1,2 moles d'iodure de triméthylsulfonium sont alors ajoutées à ce mélange qui est agité une heure à température ambiante puis 1 h 30 à 60°C et enfin 1 heure à 80°C. Après refroidissement le mélange est versé sur 3 litres d'eau glacée et le pH est amené à 6-7 avec de l'acide chlorhydrique concentré.

Un entraînement à la vapeur fournit un distillat qui est saturé par 450 g de chlorure de sodium. Après extraction par de l'éther et évaporation, le produit attendu est obtenu par distillation.
Point d'ébullition : 64-66°C (15 mm/Hg)
Rendement : 37 %

STADE B : (2,2-Dicyclopropyléthylidényl)-hydrazino-2-imidazoline

50 mmoles du produit obtenu au stade précédent et 50 mmoles d'iodhydrate de 2-hydrazino-2-imidazoline sont mis en réaction dans 40 ml d'éthanol. Après 5 heures de reflux, le solvant est évaporé. Le résidu est repris par 20 ml d'eau et 7 ml de soude 10N.

Après extraction par trois fois 50 ml de chlorure de méthylène, séchage et évaporation du solvant, le produit attendu est obtenu après recristallisation dans l'eau.
Rendement : 78 %
Point de fusion : 124-127°C

Microanalyse élémentaire :

| | C % | H % | N % |
|---|---|---|---|
| calculé | 64,05 | 8,80 | 27,16 |
| trouvé | 63,75 | 8,75 | 27,15 |

**EXEMPLE 2 : (2,2-Dicyclopropyléthylidényl) aminoguanidine, chlorhydrate**

Le produit attendu est obtenu en procédant comme dans l'exemple 1 mais en remplaçant au stade B l'iodhydrate de 2-hydrazino-2-imidazoline par le chlorhydrate d'aminoguanidine et en laissant 15 minutes à reflux.

Après refroidissement et évaporation du solvant le résidu est recristallisé dans de l'acétonitrile.
Rendement : 65 %
Point de fusion : 168-170°C

Microanalyse élémentaire :

| | C % | H % | N % | Cl % |
|---|---|---|---|---|
| calculé | 49,88 | 7,91 | 25,85 | 16,36 |
| trouvé | 50,22 | 8,14 | 26,17 | 16,38 |

**EXEMPLE 3 : 2,2-Dicyclopropyléthylamino-2-imidazoline, iodhydrate**

Le stade A est identique au stade A de l'exemple 1.

STADE B : Dicyclopropylacétaldéhyde-oxime

520 mmoles de produit obtenu au stade précédent en solution dans 60 ml d'éthanol sont versées rapidement sur une solution contenant 520 mmoles de chlorhydrate d'hydroxylamine et 27 mmoles de carbonate de sodium dans 100 ml d'eau. L'ensemble est porté à reflux 30 minutes puis abandonné 2 heures à température ambiante.

La phase aqueuse est saturée de chlorure de sodium après décantation et extraite 3 fois par 60 ml d'éther. Les phases organiques sont réunies, séchées et évaporées. Le produit attendu est obtenu par distillation.
Point d'ébullition : 110-112°C (15 mm Hg)
Rendement : 85 %

STADE C : 2,2-Dicyclopropyléthylamine, chlorhydrate

540 mmoles du produit obtenu au stade précédent en solution dans 140 ml d'éther anhydre sont versées sur une suspension contenant 860 mmoles d'hydrure de lithium-aluminium dans 1,4 litre d'éther anhydre.

Après 3 heures de reflux, la réaction est hydrolysée par 20 ml d'eau puis 17 ml de soude à 20 % et enfin par 76 ml d'eau.

Le précipité obtenu est filtré et rincé à l'éther . Le produit attendu, sous forme de base est obtenu après évaporation du solvant. Il est transformé en chlorhydrate par action de l'éther chlorhydrique.
Rendement : 67 %
Point de fusion : 140-142°C

Microanalyse élémentaire :

| | C % | H % | N % | Cl % |
|---|---|---|---|---|
| calculé | 59,43 | 9,99 | 8,68 | 21,94 |
| trouvé | 59,42 | 9,65 | 8,54 | 22,04 |

STADE D : 2,2-Dicyclopropyléthylamino-2-imidazoline, iodhydrate

60 mmoles du produit obtenu au stade précédent et 60 mmoles d'iodhydrate de 2-méthylthioimidazoline sont portées à 100°C dans 30 ml de diméthylformamide, sous atmosphère d'azote, pendant 8 heures.

Le produit attendu est obtenu après évaporation du solvant et cristallisation à froid.
Rendement : 74 %
Point de fusion : 87-93°C

Microanalyse élémentaire :

| | C % | H % | N % |
|---|---|---|---|
| calculé | 41,15 | 6,28 | 13,09 |
| trouvé | 40,98 | 6,35 | 13,08 |

**EXEMPLE 4 : (Dicyclopropylméthylène) hydrazino-2-imidazoline**

En procédant comme au stade B de l'exemple 1 mais en remplaçant le dicyclopropylacétaldéhyde par la

dicyclopropylcétone, et en laissant la réaction à reflux une nuit, on obtient le produit attendu.
Solvant de recristallisation : acétonitrile
Rendement : 70 %
Point de fusion : 120-124°C

Microanalyse élémentaire :

| | C % | H % | N % |
|---|---|---|---|
| calculé | 62,47 | 8,39 | 29,12 |
| trouvé | 62,40 | 8,30 | 28,91 |

**EXEMPLE 5 : 2-Cyclopentyl-3,3,3-trifluoropropylidénylaminoguanidine, chlorhydrate**

STADE A : Cyclopentyltrifluorométhylcétone

Une suspension de trifluoroacétate de sodium, préparée en additionnant lentement, à 0°C, 0,5 mole d'acide trifluoroacétique en solution dans le THF à 0,5 mole d'hydrure de sodium en suspension dans le THF, est ajoutée à un réactif organomagnésien lui-même préparé par addition, goutte à goutte, en entretenant le reflux, de 0,5 mole de bromure de cyclopentyle en solution dans 400 ml d'éther éthylique sur 13,4 g de tournures de magnésium placées dans 50 ml d'éther éthylique anhydre avec un cristal d'iode, le reflux étant maintenu une heure.

La suspension blanche ainsi obtenue est portée à reflux 2 heures 30 et maintenue à température ambiante une nuit.

Le mélange est alors hydrolysé, à 0°C, par 200 ml d'acide chlorhydrique 6N et 250 ml d'eau.

La phase organique est alors lavée par de l'eau, puis par une solution bicarbonatée à 10 % et à nouveau par de l'eau. Après séchage et évaporation des solvants, le produit attendu est obtenu par distillation. Point d'ébullition : 60-65°C (150 mm/Hg)

STADE B : 1-Méthoxy-2-cyclopentyl-3,3,3-trifluoropropène

A une solution de diisopropylamidure de lithium (préparée par addition, à -10°C, pendant 30 minutes, de 400 ml de n-butyllithium (1,6M) dans l'hexane sur une solution contenant 78,5 ml de diisopropylamine fraichement distillée dans 120 ml de tétrahydrofurane) est ajoutée une solution contenant 211 g de chlorure de méthoxyméthyltriphénylphosphonium dans 120 ml de tétrahydrofurane anhydre en maintenant la température en dessous de 0°C.

Une solution contenant 64 g du produit obtenu au stade précédent dans 120 ml de tétrahydrofurane est alors additionnée au mélange précédent à une température inférieure à 0°C pendant 30 minutes.

L'ensemble est agité à température ambiante pendant 72 heures, puis filtré. Le solvant est évaporé et le produit attendu est obtenu après distillation du résidu sous vide.
Rendement : 50 %
Point d'ébullition : 80-87°C (40 mm/Hg)

STADE C : Cyclopentyltrifluorométhylacétaldéhyde

A une suspension de 180 mmoles du produit obtenu au stade précédent dans 60 ml d'eau sont additionnées, goutte à goutte, sous agitation 15 ml d'acide sulfurique concentré à une température comprise entre 0 et 3°C.

L'ensemble est maintenu sous agitation pendant 4 heures à température ambiante. Après extraction par 3 fois 50 ml d'éther, lavage des phases organiques par de l'eau, séchage et évaporation du solvant, le produit attendu est obtenu par distillation sous vide.
Point d'ébullition : 72-74°C (50 mm/Hg)

STADE D : 2-Cyclopropyl-3,3,3-trifluoropropylidénylaminoguanidine, chlorhydrate

Une solution contenant 5,6 mmoles du produit obtenu au stade précédent et 5,6 mmoles de chlorhydrate d'aminoguanidine dans 10 ml d'éthanol anhydre est portée à reflux pendant 30 minutes. Après refroidissement et évaporation du solvant, on obtient le produit attendu qui est recristallisé dans le toluène.

Rendement : 60 %
Point de fusion : 107-109°C

Microanalyse élémentaire :

| | C % | H % | N % | Cl % |
|---|---|---|---|---|
| calculé | 39,64 | 5,91 | 20,54 | 13,00 |
| trouvé | 39,47 | 5,88 | 20,66 | 13,14 |

**EXEMPLE 6 : 2-Phényl-3,3,3-trifluoropropylidénylaminoguanidine, chlorhydrate**

En procédant comme dans l'exemple 5 (stades B à D) mais en remplaçant au stade B, la cyclopentyltrifluorométhylcétone par la phényltrifluorométhylcétone, on obtient le produit attendu.
Rendement :
Point de fusion : 117-120°C

Microanalyse élémentaire :

| | C % | H % | N % | Cl % |
|---|---|---|---|---|
| calculé | 42,79 | 4,31 | 19,96 | 12,63 |
| trouvé | 42,93 | 4,35 | 19,83 | 12,45 |

**EXEMPLE 7 : 2-(2-Phényl-3,3,3-trifluoro-1-propylamino)imidazoline, fumarate**

STADE A : 1-Méthoxyimino-2-phényl-3,3,3-trifluoropropane

Un mélange contenant 40 mmoles de phényltrifluorométhylacétaldéhyde, 43 mmoles de chlorhydrate de O-méthylhydroxylamine, 100 ml de pyridine distillée dans 100 ml d'éthanol anhydre est porté à reflux pendant 2 heures. Après évaporation du solvant à pression réduite à une température inférieure à 25°C, le résidu est traité par 50 ml d'eau et extrait par 3 fois 50 ml d'éther.

Le produit attendu est alors obtenu après séchage et évaporation de la phase éthérée, et distillation sous vide.
Rendement : 96 %
Point d'ébullition : 108-110°C (22 mm/Hg)

STADE B : 2-Phényl-3,3,3-trifluoropropylamine

A une solution contenant 38 mmoles du produit obtenu au stade précédent dans 30 ml de tétrahydrofurane anhydre, refroidie à 5°C, est additionnée 115 ml d'une solution molaire de diborane dans du tétrahydrofurane en maintenant la température au dessous de 10°C.

L'ensemble est alors porté à reflux 3 heures, refroidi à 20°C puis traité par 40 ml de méthanol et à nouveau porté à reflux 3 heures. Après refroidissement, les solvants sont évaporés, à pression réduite, à une température inférieure à 25°C.

Le résidu est repris par 30 ml d'éther, filtré, traité par une solution d'éther chlorhydrique. Le chlorhydrate ainsi obtenu est alors traité par de la soude et le produit attendu est obtenu après extraction par de l'éther, séchage et évaporation du solvant et distillation sous vide.
Rendement : 85 %
Point d'ébullition : 105-110°C (18 mm/Hg)

STADE C : 2-(2-Phényl-3,3,3-trifluoro-1-propylamino) imidazoline, fumarate

14,5 mmoles du produit obtenu au stade précédent et 14,5 mmoles d'iodhydrate de 2-mercapto-4, 5-dihydro imidazole dans 20 ml de diméthylformamide anhydre sont portées à reflux pendant 12 heures.

Après refroidissement et évaporation du solvant, le résidu est dissous dans 2 ml d'eau, traité par 2 ml de soude (35 %) et extrait par 3 x 15 ml d'éther. Après lavage des phases éthérées par de l'eau, séchage et évaporation, le produit attendu est obtenu sous forme de fumarate par traitement de l'huile par 10 mmoles d'acide fumarique dans 50 ml d'éthanol, évaporation et recristallisation dans de l'isopropanol.
Rendement : 53 %
Point de fusion : 132-134°C

Microanalyse élémentaire :

| | C % | H % | N % |
|---|---|---|---|
| calculé | 51,48 | 4,86 | 11,26 |
| trouvé | 51,17 | 4,79 | 11,19 |

ETUDE PHARMACOLOGIQUE DES DERIVES DE L'INVENTION

**EXEMPLE 8 : Test d'affinité in vitro pour les récepteurs endazoliniques, $\alpha_1$ et $\alpha_2$ et 5-HT**

Les tests d'affinité in vitro pour les récepteurs endazoliques ont été réalisés selon une technique décrite par A. PARINI et Coll. (Biochem. and Biophys. Res. Comm. 147(3), 1055, 1987) et selon des techniques classiques de binding pour les autres récepteurs.

Les résultats de ces études montrent, en particulier, que le composé de l'exemple 2 possède un $K_i$ de l'ordre de $10^{-8}$ M vis-à-vis des récepteurs endazoliniques et de l'ordre de :
- $2.10^{-6}$ vis-à-vis des récepteur $\alpha_2$ adrénergiques
- supérieur à $10^{-4}$ vis-à-vis des récepteurs $\alpha_1$ adrénergiques
- $10^{-5}$ vis-à-vis des récepteurs $5\text{-HT}_{1A}$
- $10^{-4}$ vis-à-vis des récepteurs $5\text{-HT}_{1B}$
- $2.10^{-5}$ vis-à-vis des récepteurs $5\text{-HT}_2$
- $2.10^{-6}$ vis-à-vis des récepteurs $5\text{-HT}_3$

Ces résultats montrent non seulement l'affinité du composé pour les récepteurs endazoliques mais également la grande sélectivité vis-à-vis des autres récepteurs testés.

COMPOSITION PHARMACEUTIQUE

**EXEMPLE 9 : Comprimé : formule de préparation pour 1000 comprimés dosés à 2 mg**

| | |
|---|---|
| (2,2-Dicyclopropyléthylidényl) aminoguanidine | 2 g |
| Hydroxypropyl cellulose | 2 g |
| Amidon de blé | 10 g |
| Lactose | 100 g |
| Stéarate de magnésium | 3 g |
| Talc | 3 g |

**Revendications**

**Revendications pour les Etats contractants suivants : AT, BE, CH, DE, DK, FR, GB, IT, LI, LU, NL, SE**

1. Composés de formule générale (I) :

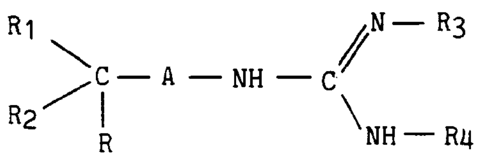

(I)

dans laquelle :

$R_1$ représente - un groupement phényle éventuellement substitué par un ou plusieurs atomes d'halogène ou groupements alkyle ($C_1$-$C_6$) linéaire ou ramifié, hydroxy, alkoxy ($C_1$-$C_6$) linéaire ou ramifié,
- un groupement cycloalkyle contenant de 3 à 7 atomes de carbone,

$R_2$ représente un groupement trifluorométhyle ou un groupement cycloalkyle contenant de 3 à 4 atomes de carbone,

A représente un groupement $-CH_2-$, -CH= N-, = N-,

$R_3$ et $R_4$ identiques ou différents représentent un atome d'hydrogène, un groupement alkyle ($C_1$-$C_6$) linéaire ou ramifié, ou forment avec les atomes d'azote auxquels ils sont attachés un hétérocycle à 5 ou 6 chaînons,

R représente soit un atome d'hydrogène dans le cas où A représente un groupement $-CH_2-$ ou -CH =N-, soit une liaison du groupement A quand celui-ci est représenté par = N-, à la condition toutefois que, lorsque A représente un groupement =N- et $R_1$ un groupement phényle éventuellement substitué, $R_2$ ne représente pas simultanément un groupement cycloalkyle ($C_3$-$C_4$),

leurs énantiomères et épimères ainsi que leurs sels d'addition à un acide pharmaceutiquement acceptable.

2. Composés selon la revendication 1 tels que $R_2$ représente un groupement trifluorométhyle, leurs énantiomères et épimères ainsi que leurs sels d'addition à un acide pharmaceutiquement acceptable.

3. Composés selon la revendication 1 tels que $R_1$ et $R_2$ représentent chacun un groupement cyclopropyle, leurs énantiomères et épimères ainsi que leurs sels d'addition à un acide pharmaceutiquement acceptable.

4. Composés selon la revendication 1 tels que A représente un groupement -CH = N -, leurs énantiomères et épimères ainsi que leurs sels d'addition à un acide pharmaceutiquement acceptable.

5. Composés selon la revendication 1 tels que $R_3$ et $R_4$ représentent chacun un atome d'hydrogène, leurs énantiomères et épimères ainsi que leurs sels d'addition à un acide pharmaceutiquement acceptable.

6. Composés selon la revendication 1 tels que $R_3$ et $R_4$ forment avec les atomes d'azote auxquels ils sont attachés un cycle imidazoline, leurs énantiomères et épimères ainsi que leurs sels d'addition à un acide pharmaceutiquement acceptable.

7. Composé selon l'une quelconque des revendications 1, 3, 4 ou 5 qui est la (2,2-dicyclopropyléthylidényl) aminoguanidine, et ses sels d'addition à un acide pharmaceutiquement acceptable.

8. Procédé de préparation des composés de formule (I) caractérisé en ce que l'on utilise comme produit de départ une cétone de formule (II) :

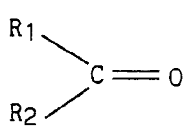

(II)

dans laquelle $R_1$ et $R_2$ ont la même signification que dans la formule (I)
que l'on soumet
1/ soit
à l'action d'un composé de formule (III) dans un solvant organique

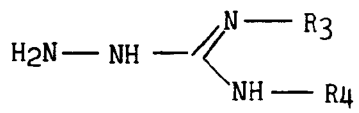

(III)

dans laquelle $R_3$ et $R_4$ ont la même signification que dans la formule (I)
pour conduire à un composé de formule (I/a), cas particulier des composés de formule (I)

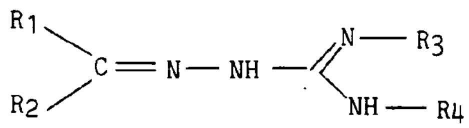

(I/a)

dans laquelle $R_1$, $R_2$, $R_3$ et $R_4$ ont la même signification que dans la formule (I), A représente un groupement =N- et R une liaison de ce groupement,

2/ soit

à l'action du chlorure de méthoxyméthyltriphénylphosphonium pour conduire à l'éther d'énol de formule (IV)

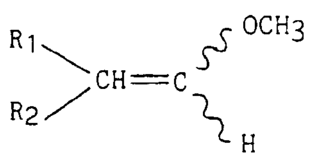

(IV)

dans laquelle $R_1$ et $R_2$ ont la même signification que dans la formule (I)
que l'on hydrolyse en milieu acide pour conduire à l'aldéhyde de formule (V)

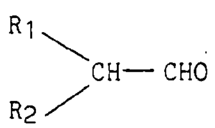

(V)

dans laquelle $R_1$ et $R_2$ ont la même signification que dans la formule (I),
que l'on soumet

ou bien

à l'action d'un composé de formule (III)

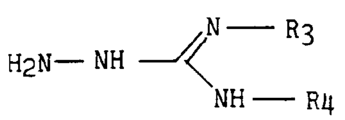

(III)

dans laquelle $R_3$ et $R_4$ ont la même signification que dans la formule (I)
pour conduire à un composé de formule (I/c), cas particulier des composés de formule (I)

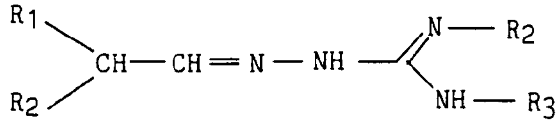

(I/c)

dans laquelle $R_1$, $R_2$, $R_3$, $R_4$ ont la même signification que dans la formule (I), A représente un groupement -CH = N- et R un atome d'hydrogène,

ou bien

à l'action d'un composé de formule (VI) en présence de pyridine

$$H_2N - O - R' \qquad (VI)$$

dans laquelle R′ représente un atome d'hydrogène ou un groupement alkyle ($C_1$-$C_6$), pour conduire à un composé de formule (VII)

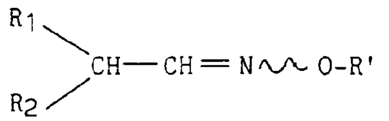

(VII)

dans laquelle $R_1$, $R_2$ et R′ ont la même signification que précédemment que l'on réduit par hydrogénation catalytique ou en présence d'un hydrure mixte de métal alcalin pour conduire à une amine de formule (VIII)

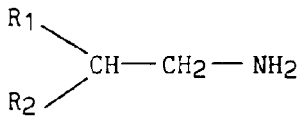

(VIII)

dans laquelle $R_1$ et $R_2$ ont même signification que dans la formule (I), que l'on soumet à l'action d'un composé de formule (IX)

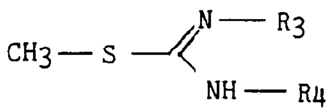

(IX)

dans laquelle $R_3$ et $R_4$ ont la même signification que dans la formule (I) pour conduire à un composé de formule (I/d), cas particulier des composés de formule (I)

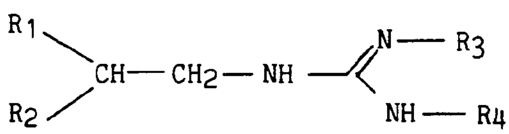

(I/d)

dans laquelle $R_1$, $R_2$, $R_3$, $R_4$ ont la même signification que dans la formule (I), A représente un groupement $-CH_2-$ et R un atome d'hydrogène,
composés de formule (I/a), (I/c) et (I/d) qui constituent l'ensemble des composés de formule (I), que l'on purifie, le cas échéant, par des techniques classiques de purification, dont on sépare, si on le souhaite, les isomères par des techniques classiques de séparation et que l'on transforme, si nécessaire, en leurs sels d'addition à un acide pharmaceutiquement acceptable.

**9.** Compositions pharmaceutiques contenant comme principe actif au moins un composé selon l'une quelconque des revendications 1 à 7, seul ou en combinaison avec un ou plusieurs excipients ou véhicules inertes non toxiques, pharmaceutiquement acceptables.

**10.** Compositions pharmaceutiques selon la revendication 9 pour l'utilisation dans le traitement de la dépression, de l'hypertension artérielle, des troubles vasculaires, du diabète, de l'obésité et des maladies pulmonaires.

**Revendications pour l'Etat contractant suivant : ES**

**1.** Procédé de préparation des composés de formule générale (I) :

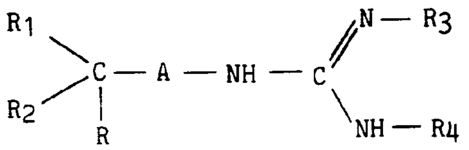

(I)

dans laquelle :

$R_1$ représente - un groupement phényle éventuellement substitué par un ou plusieurs atomes d'halogène ou groupements alkyle ($C_1$-$C_6$) linéaire ou ramifié, hydroxy, alkoxy ($C_1$-$C_6$) linéaire ou ramifié,
- un groupement cycloalkyle contenant de 3 à 7 atomes de carbone,

$R_2$ représente un groupement trifluorométhyle ou un groupement cycloalkyle contenant de 3 à 4 atomes de carbone,

A représente un groupement $-CH_2-$, -CH = N-, = N-,

$R_3$ et $R_4$ identiques ou différents représentent un atome d'hydrogène, un groupement alkyle ($C_1$-$C_6$) linéaire ou ramifié, ou forment avec les atomes d'azote auxquels ils sont attachés un hétérocycle à 5 ou 6 chaînons,

R représente soit un atome d'hydrogène dans le cas où A représente un groupement $-CH_2-$ ou -CH = N-, soit une liaison du groupement A quand celui-ci est représenté par = N-, à la condition toutefois que, lorsque A représente un groupement =N- et $R_1$ un groupement phényle éventuellement substitué, $R_2$ ne représente pas simultanément un groupement cycloalkyle ($C_3$-$C_4$),

leurs énantiomères et épimères ainsi que leurs sels d'addition à un acide pharmaceutiquement acceptable,
caractérisé en ce que l'on utilise comme produit de départ une cétone de formule (II) :

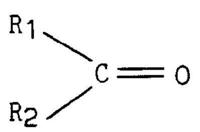

(II)

dans laquelle $R_1$ et $R_2$ ont la même signification que dans la formule (I)
que l'on soumet
1/ soit
à l'action d'un composé de formule (III) dans un solvant organique

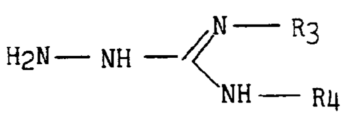

(III)

dans laquelle $R_3$ et $R_4$ ont la même signification que dans la formule (I)
pour conduire à un composé de formule (I/a), cas particulier des composés de formule (I)

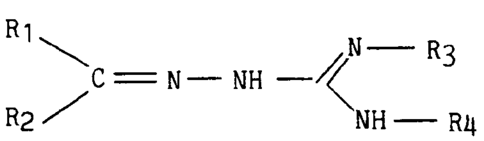

(I/a)

dans laquelle $R_1$, $R_2$, $R_3$ et $R_4$ ont la même signification que dans la formule (I), A représente un groupement =N- et R une liaison de ce groupement,
2/ soit
à l'action du chlorure de méthoxyméthyltriphénylphosphonium pour conduire à l'éther d'énol de formule (IV)

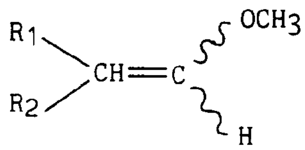 (IV)

dans laquelle $R_1$ et $R_2$ ont la même signification que dans la formule (I) que l'on hydrolyse en milieu acide pour conduire à l'aldéhyde de formule (V)

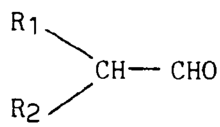 (V)

dans laquelle $R_1$ et $R_2$ ont la même signification que dans la formule (I), que l'on soumet

<u>ou bien</u>

à l'action d'un composé de formule (III)

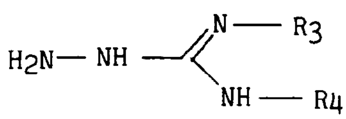 (III)

dans laquelle $R_3$ et $R_4$ ont la même signification que dans la formule (I) pour conduire à un composé de formule (I/c), cas particulier des composés de formule (I)

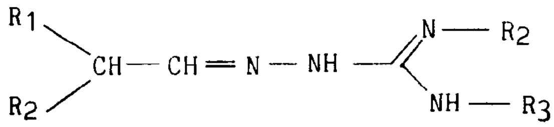 (I/c)

dans laquelle $R_1$, $R_2$, $R_3$, $R_4$ ont la même signification que dans la formule (I), A représente un groupement -CH = N- et R un atome d'hydrogène,

<u>ou bien</u>

à l'action d'un composé de formule (VI) en présence de pyridine

$H_2N$ - O - R' (VI)

dans laquelle R' représente un atome d'hydrogène ou un groupement alkyle ($C_1$-$C_6$), pour conduire à un composé de formule (VII)

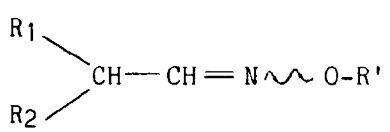 (VII)

dans laquelle $R_1$, $R_2$ et R' ont la même signification que précédemment que l'on réduit par hydrogénation catalytique ou en présence d'un hydrure mixte de métal alcalin pour conduire à une amine de formule (VIII)

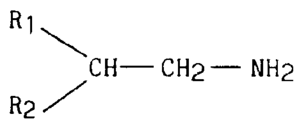 (VIII)

dans laquelle $R_1$ et $R_2$ ont même signification que dans la formule (I), que l'on soumet à l'action d'un composé de formule (IX)

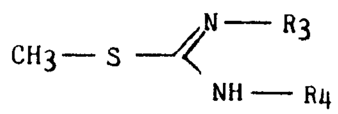

(IX)

dans laquelle $R_3$ et $R_4$ ont la même signification que dans la formule (I) pour conduire à un composé de formule (I/d), cas particulier des composés de formule (I)

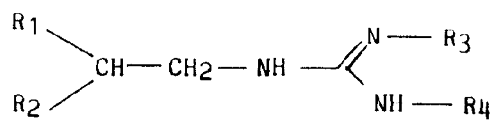

(I/d)

dans laquelle $R_1$, $R_2$, $R_3$, $R_4$ ont la même signification que dans la formule (I), A représente un groupement $-CH_2-$ et R un atome d'hydrogène, composés de formule (I/a), (I/c) et (I/d) qui constituent l'ensemble des composés de formule (I), que l'on purifie, le cas échéant, par des techniques classiques de purification, dont on sépare, si on le souhaite, les isomères par des techniques classiques de séparation et que l'on transforme, si nécessaire, en leurs sels d'addition à un acide pharmaceutiquement acceptable.

2. Procédé de préparation selon la revendication 1 des composés tels que $R_2$ représente un groupement trifluorométhyle, leurs énantiomères et épimères ainsi que leurs sels d'addition à un acide pharmaceutiquement acceptable.

3. Procédé de préparation selon la revendication 1 des composés tels que $R_1$ et $R_2$ représentent chacun un groupement cyclopropyle, leurs énantiomères et épimères ainsi que leurs sels d'addition à un acide pharmaceutiquement acceptable.

4. Procédé de préparation selon la revendication 1 des composés tels que A représente un groupement -CH = N -, leurs énantiomères et épimères ainsi que leurs sels d'addition à un acide pharmaceutiquement acceptable.

5. Procédé de préparation selon la revendication 1 des composés tels que $R_3$ et $R_4$ représentent chacun un atome d'hydrogène, leurs énantiomères et épimères ainsi que leurs sels d'addition à un acide pharmaceutiquement acceptable.

6. Procédé de préparation selon la revendication 1 des composés tels que $R_3$ et $R_4$ forment avec les atomes d'azote auxquels ils sont attachés un cycle imidazoline, leurs énantiomères et épimères ainsi que leurs sels d'addition à un acide pharmaceutiquement acceptable.

7. Procédé de préparation selon l'une quelconque des revendications 1, 3, 4 ou 5 du composé qui est la (2,2-dicyclopropyléthylidényl) aminoguanidine, et ses sels d'addition à un acide pharmaceutiquement acceptable.

**Revendications pour l'Etat contractant suivant : GR**

1. Procédé de préparation des composés de formule générale (I) :

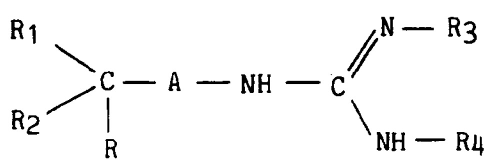

(I)

dans laquelle :

$R_1$ représente - un groupement phényle éventuellement substitué par un ou plusieurs atomes d'halogène ou groupements alkyle ($C_1$-$C_6$) linéaire ou ramifié, hydroxy, alkoxy ($C_1$-$C_6$) linéaire ou ramifié,
- un groupement cycloalkyle contenant de 3 à 7 atomes de carbone,

$R_2$ représente un groupement trifluorométhyle ou un groupement cycloalkyle contenant de 3 à 4 atomes de carbone,

A représente un groupement $-CH_2-$, $-CH=N-$, $=N-$,

$R_3$ et $R_4$ identiques ou différents représentent un atome d'hydrogène, un groupement alkyle ($C_1$-$C_6$) linéaire ou ramifié, ou forment avec les atomes d'azote auxquels ils sont attachés un hétérocycle à 5 ou 6 chaînons,

R représente soit un atome d'hydrogène dans le cas où A représente un groupement $-CH_2-$ ou $-CH=N-$, soit une liaison du groupement A quand celui-ci est représenté par $=N-$, à la condition toutefois que, lorsque A représente un groupement $=N-$ et $R_1$ un groupement phényle éventuellement substitué, $R_2$ ne représente pas simultanément un groupement cycloalkyle ($C_3$-$C_4$),

leurs énantiomères et épimères ainsi que leurs sels d'addition à un acide pharmaceutiquement acceptable,

caractérisé en ce que l'on utilise comme produit de départ une cétone de formule (II) :

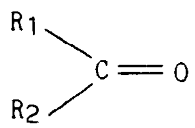

(II)

dans laquelle $R_1$ et $R_2$ ont la même signification que dans la formule (I)
que l'on soumet
1/ soit
à l'action d'un composé de formule (III) dans un solvant organique

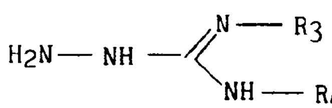

(III)

dans laquelle $R_3$ et $R_4$ ont la même signification que dans la formule (I)
pour conduire à un composé de formule (I/a), cas particulier des composés de formule (I)

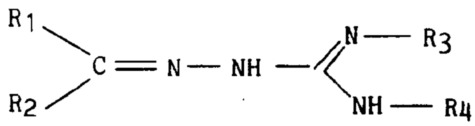

(I/a)

dans laquelle $R_1$, $R_2$, $R_3$ et $R_4$ ont la même signification que dans la formule (I), A représente un groupement $=N-$ et R une liaison de ce groupement,
2/ soit
à l'action du chlorure de méthoxyméthyltriphénylphosphonium pour conduire à l'éther d'énol de formule (IV)

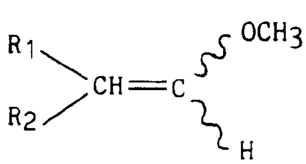

(IV)

dans laquelle $R_1$ et $R_2$ ont la même signification que dans la formule (I) que l'on hydrolyse en milieu acide pour conduire à l'aldéhyde de formule (V)

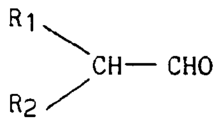

(V)

dans laquelle $R_1$ et $R_2$ ont la même signification que dans la formule (I), que l'on soumet

<u>ou bien</u>

à l'action d'un composé de formule (III)

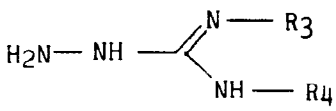

(III)

dans laquelle $R_3$ et $R_4$ ont la même signification que dans la formule (I) pour conduire à un composé de formule (I/c), cas particulier des composés de formule (I)

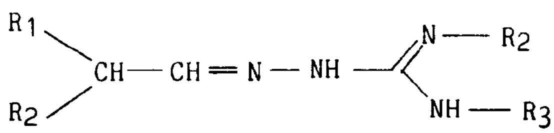

(I/c)

dans laquelle $R_1$, $R_2$, $R_3$, $R_4$ ont la même signification que dans la formule (I), A représente un groupement -CH =N- et R un atome d'hydrogène,

<u>ou bien</u>

à l'action d'un composé de formule (VI) en présence de pyridine

$H_2N - O - R'$ (VI)

dans laquelle R' représente un atome d'hydrogène ou un groupement alkyle ($C_1$-$C_6$), pour conduire à un composé de formule (VII)

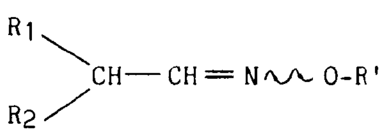

(VII)

dans laquelle $R_1$, $R_2$ et R' ont la même signification que précédemment que l'on réduit par hydrogénation catalytique ou en présence d'un hydrure mixte de métal alcalin pour conduire à une amine de formule (VIII)

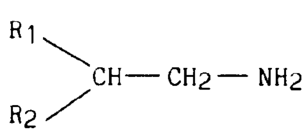

(VIII)

dans laquelle $R_1$ et $R_2$ ont même signification que dans la formule (I), que l'on soumet à l'action d'un composé de formule (IX)

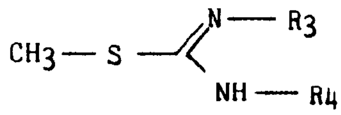

(IX)

dans laquelle $R_3$ et $R_4$ ont la même signification que dans la formule (I)
pour conduire à un composé de formule (I/d), cas particulier des composés de formule (I)

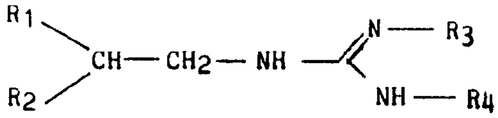

(I/d)

dans laquelle $R_1$, $R_2$, $R_3$, $R_4$ ont la même signification que dans la formule (I), A représente un groupement $-CH_2-$ et R un atome d'hydrogène,
composés de formule (I/a), (I/c) et (I/d) qui constituent l'ensemble des composés de formule (I), que l'on purifie, le cas échéant, par des techniques classiques de purification, dont on sépare, si on le souhaite, les isomères par des techniques classiques de séparation et que l'on transforme, si nécessaire, en leurs sels d'addition à un acide pharmaceutiquement acceptable.

2. Procédé de préparation selon la revendication 1 des composés tels que $R_2$ représente un groupement trifluorométhyle, leurs énantiomètre et épimères ainsi que leurs sels d'addition à un acide pharmaceutiquement acceptable.

3. Procédé de préparation selon la revendication 1 des composés tels que $R_1$ et $R_2$ représentent chacun un groupement cyclopropyle, leurs énantiomères et épimères ainsi que leurs sels d'addition à un acide pharmaceutiquement acceptable.

4. Procédé de préparation selon la revendication 1 des composés tels que A représente un groupement $-CH = N-$, leurs énantiomètres et épimères ainsi que leurs sels d'addition à un acide pharmaceutiquement acceptable.

5. Procédé de préparation selon la revendication 1 des composés tels que $R_3$ et $R_4$ représentent chacun un atome d'hydrogène, leurs énantiomères et épimères ainsi que leurs sels d'addition à un acide pharmaceutiquement acceptable.

6. Procédé de préparation selon la revendication 1 des composés tels que $R_3$ et $R_4$ forment avec les atomes d'azote auxquels ils sont attachés un cycle imidazoline, leurs énantiomères et épimères ainsi que leurs sels d'addition à un acide pharmaceutiquement acceptable.

7. Procédé de préparation selon l'une quelconque des revendications 1, 3, 4 ou 5 du composé qui est la (2,2-dicyclopropyléthylidényl) aminoguanidine, et ses sels d'addition à un acide pharmaceutiquement acceptable.

## Patentansprüche

### Patentansprüche für folgende Vertragsstaaten : AT, BE, CH, DE, DK, FR, GB, IT, LI, LU, NL, SE

1. Verbindungen der allgemeinen Formel (I):

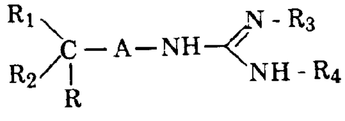

(I)

in der

| | |
|---|---|
| $R_1$ | - eine Phenylgruppe, die gegebenenfalls durch eines oder mehrere Halogenatome, geradkettige oder verzweigte $C_1$-$C_6$-Alkylgruppen, Hydroxylgruppen oder geradkettige oder verzweigte $C_1$-$C_6$-Alkoxygruppen substituiert ist, oder<br>- eine Cycloalkylgruppe mit 3 bis 7 Kohlenstoffatomen, |
| $R_2$ | eine Trifluormethylgruppe oder eine Cycloalkylgruppe mit 3 bis 4 Kohlenstoffatomen, |
| A | eine Gruppe der Formel $-CH_2-$, -C=N-, =N-, |
| $R_3$ und $R_4$, | die gleichartig oder verschieden sein können, Wasserstoffatome, geradkettige oder verzweigte $C_1$-$C_6$-Alkylgruppen oder gemeinsam mit den Stickstoffatomen, an die sie gebunden sind. einen Heterocyclus mit 5 oder 6 Kettengliedern und |
| R | entweder ein Wasserstoffatom dann, wenn A eine Gruppe der Formel $-CH_2-$ oder -CH=N- darstellt, oder eine Bindung der Gruppe A dann, wenn diese eine Gruppe der Formel =N- darstellt,<br>mit der Maßgabe bedeuten, daß, wenn A eine Gruppe der Formel =N- und $R_1$ eine gegebenenfalls substituierte Phenylgruppe darstellen, $R_2$ nicht gleichzeitig eine $C_3$-$C_4$-Cycloalkylgruppe bedeutet, |

deren Enantiomere und Epimere sowie deren Additionssalze mit einer pharmazeutisch annehmbaren Säure.

2. Verbindungen nach Anspruch 1, worin $R_2$ eine Trifluormethylgruppe bedeutet, deren Enantiomere und Epimere sowie deren Additionssalze mit einer pharmazeutisch annehmbaren Säure.

3. Verbindungen nach Anspruch 1, worin $R_1$ und $R_2$ jeweils eine Cyclopropylgruppe bedeuten. deren Enantiomere und Epimere sowie deren Additionssalze mit einer pharmazeutisch annehmbaren Säure.

4. Verbindungen nach Anspruch 1, worin A eine Gruppe der Formel -CH=N- bedeutet, deren Enantiomere und Epimere sowie deren Additionssalze mit einer pharmazeutisch annehmbaren Säure.

5. Verbindungen nach Anspruch 1, worin $R_3$ und $R_4$ jeweils ein Wasserstoffatom bedeuten. deren Enantiomere und Epimere sowie deren Additionssalze mit einer pharmazeutisch annehmbaren Säure.

6. Verbindungen nach Anspruch 1, worin $R_3$ und $R_4$ mit den Stickstoffatomen, an die sie gebunden sind, einen Imidazolinring bilden, deren Enantiomere und Epimere sowie deren Additionssalze mit einer pharmazeutisch annehmbaren Säure.

7. Verbindungen nach einem der Ansprüche 1, 3, 4 oder 5, nämlich (2,2-Dicyclopropylethylidenyl)-aminoguanidin und dessen Additionssalze mit einer pharmazeutisch annehmbaren Säure.

8. Verfahren zur Herstellung der Verbindungen der Formel (1), **dadurch gekennzeichnet,** daß man als Ausgangsmaterial ein Keton der Formel (II):

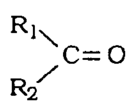

(II)

worin $R_1$ und $R_2$ die bezuglich der Formel (I) angegebenen Bedeutungen besitzen, verwendet, welches man

1/ **entweder**

der Einwirkung einer Verbindung der Formel (III)

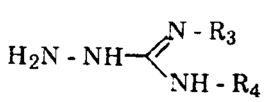

(III)

in der $R_3$ und $R_4$ die bezüglich der Formel (I) angegebenen Bedeutungen besitzen, in einem organischen Lösungsmittel unterwirft zur Bildung einer Verbindung der Formel (I/a), einem Sonderfall der Verbindungen der Formel (I)

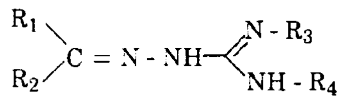 (I/a)

in der $R_1$, $R_2$, $R_3$ und $R_4$ die für die Formel (I) angegebenen Bedeutungen besitzen, A eine Gruppe der Formel =N- und R eine Bindung dieser Gruppe bedeuten,

2/ **oder**

der Einwirkung von Methoxymethyltriphenylphosphoniumchlorid unterwirft zur Bildung eines Enolethers der Formel (IV)

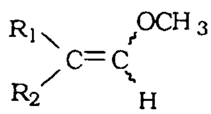 (IV)

in der $R_1$ und $R_2$ die bezüglich der Formel (I) angegebenen Bedeutungen besitzen,
welchen man in saurem Medium hydrolysiert zur Bildung eines Aldehyds der Formel (V)

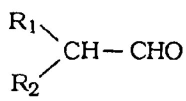 (V)

in der $R_1$ und $R_2$ die bezüglich der Formel (I) angegebenen Bedeutungen besitzen,
welchen man

<u>entweder</u>

der Einwirkung einer Verbindung der Formel (III)

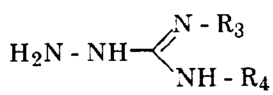 (III)

in der $R_3$ und $R_4$ die bezüglich der Formel (I) angegebenen Bedeutungen besitzen, unterwirft zur Bildung einer Verbindung der Formel (I/c), einem Sonderfall der Verbindungen der Formel (I)

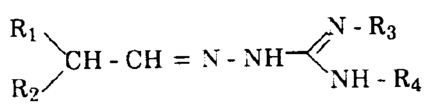 (I/c)

in der $R_1$, $R_2$, $R_3$ und $R_4$ die bezüglich der Formel (I) angegebenen Bedeutungen besitzen, und A eine Gruppe der Formel -CH=N- und R ein Wasserstoffatom bedeuten,

<u>oder</u>

der Einwirkung einer Verbindung der Formel (VI)

$H_2N$-O-R' (VI)

in der R' ein Wasserstoffatom oder eine $C_1$-$C_6$-Alkylgruppe bedeutet, in Gegenwart von Pyridin unterwirft zur Bildung einer Verbindung der Formel (VII)

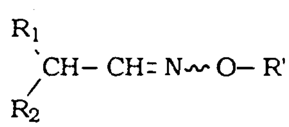 (VII)

in der $R_1$, $R_2$ und R' die oben angegebenen Bedeutungen besitzen, welche man durch katalytische Hydrierung oder in Gegenwart eines gemischten Alkalimetallhydrids reduziert zur Bildung eines Amins

der Formel (VIII)

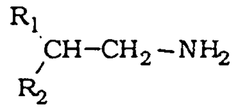

(VIII)

in der $R_1$ und $R_2$ die bezüglich der Formel (I) angegebenen Bedeutungen besitzen,
welches man der Einwirkung einer Verbindung der Formel (IX)

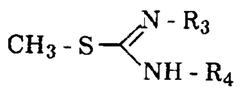

(IX)

in der $R_3$ und $R_4$ die bezüglich der Formel (I) angegebenen Bedeutungen besitzen, unterwirft, zur Bildung einer Verbindung der Formel (I/d), einem Sonderfall der Verbindungen der Formel (I)

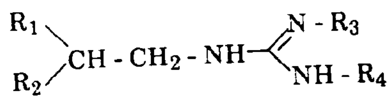

(I/d)

in der $R_1$, $R_2$, $R_3$ und $R_4$ die bezüglich der Formel (I) angegebenen Bedeutungen besitzen und A eine Gruppe der Formel $-CH_2-$ und R ein Wasserstoffatom bedeuten,
wobei die Verbindungen der Formeln (I/a), (I/c) und (I/d) gemeinsam die Verbindungen der Formel (I) darstellen, die man gegebenenfalls durch klassische Reinigungsverfahren reinigt, die man gewünschtenfalls mit Hilfe klassischer Trennverfahren in die Isomeren auftrennt und die man erforderlichenfalls in ihre Additionssalze mit einer pharmazeutisch annehmbaren Säure überführt.

9. Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Bindemitteln oder Trägermaterialien.

10. Pharmazeutische Zubereitungen nach Anspruch 9 zur Verwendung bei der Behandlung der Depression, der arteriellen Hypertension, von Gefäßstörungen, des Diabetes, der Fettsucht und von Lungenerkrankungen.

**Patentansprüche für folgenden Vertragsstaat : ES**

1. Verfahren zur Herstellung der Verbindungen der allgemeinen Formel (I):

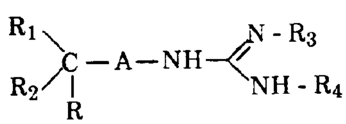

(I)

in der

$R_1$ - eine Phenylgruppe. die gegebenenfalls durch eines oder mehrere Halogenatome, geradkettige oder verzweigte $C_1$-$C_6$-Alkylgruppen, Hydroxylgruppen oder geradkettige oder verzweigte $C_1$-$C_6$-Alkoxygruppen substituiert ist, oder
- eine Cycloalkylgruppe mit 3 bis 7 Kohlenstoffatomen,

$R_2$ eine Trifluormethylgruppe oder eine Cycloalkylgruppe mit 3 bis 4 Kohlenstoffatomen,

A eine Gruppe der Formel $-CH_2-$, -C=N-, =N-,

$R_3$ und $R_4$, die gleichartig oder verschieden sein können, Wasserstoffatome, geradkettige oder verzweigte $C_1$-$C_6$-Alkylgruppen oder gemeinsam mit den Stickstoffatomen, an die sie gebunden sind, einen Heterocyclus mit 5 oder 6 Kettengliedern und

R entweder ein Wasserstoffatom dann, wenn A eine Gruppe der Formel $-CH_2-$ oder -

CH=N- darstellt, oder eine Bindung der Gruppe A dann, wenn diese eine Gruppe der Formel =N- darstellt,

mit der Maßgabe bedeuten, daß, wenn A eine Gruppe der Formel =N- und $R_1$ eine gegebenenfalls substituierte Phenylgruppe darstellen, $R_2$ nicht gleichzeitig eine $C_3$-$C_4$-Cycloalkylgruppe bedeutet,

deren Enantiomere und Epimere sowie ihrer Additionssalze mit einer pharmazeutisch annehmbaren Säure, **dadurch gekennzeichnet,** daß man als Ausgangsmaterial ein Keton der Formel (II):

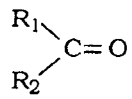

(II)

worin $R_1$ und $R_2$ die bezüglich der Formel (I) angegebenen Bedeutungen besitzen, verwendet, welches man

1/ **entweder**

der Einwirkung einer Verbindung der Formel (III)

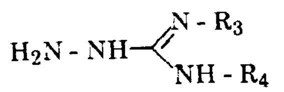

(III)

in der $R_3$ und $R_4$ die bezüglich der Formel (I) angegebenen Bedeutungen besitzen, in einem organischen Losungsmittel unterwirft zur Bildung einer Verbindung der Formel (I/a), einem Sonderfall der Verbindungen der Formel (I)

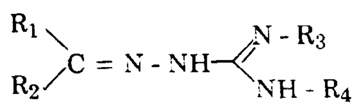

(I/a)

in der $R_1$, $R_2$, $R_3$ und $R_4$ die für die Formel (I) angegebenen Bedeutungen besitzen, A eine Gruppe der Formel =N- und R eine Bindung dieser Gruppe bedeuten,

2/ **oder**

der Einwirkung von Methoxymethyltriphenylphosphoniumchlorid unterwirft zur Bildung eines Enolethers der Formel (IV)

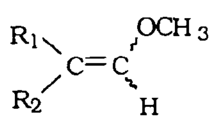

(IV)

in der $R_1$ und $R_2$ die bezüglich der Formel (I) angegebenen Bedeutungen besitzen,
welchen man in saurem Medium hydrolysiert zur Bildung eines Aldehyds der Formel (V)

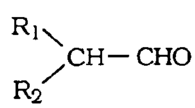

(V)

in der $R_1$ und $R_2$ die bezüglich der Formel (I) angegebenen Bedeutungen besitzen,
welchen man

entweder

der Einwirkung einer Verbindung der Formel (III)

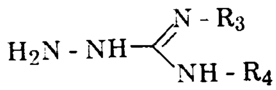 (III)

in der $R_3$ und $R_4$ die bezüglich der Formel (I) angegebenen Bedeutungen besitzen, unterwirft zur Bildung einer Verbindung der Formel (I/c), einem Sonderfall der Verbindungen der Formel (I)

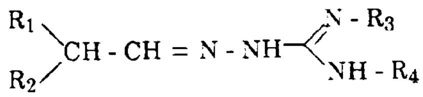 (I/c)

in der $R_1$, $R_2$ $R_3$ und $R_4$ die bezüglich der Formel (I) angegebenen Bedeutungen besitzen. und A eine Gruppe der Formel -CH=N- und R ein Wasserstoffatom bedeuten,

<u>oder</u>

der Einwirkung einer Verbindung der Formel (VI)

$H_2N$-O-R' (VI)

in der R' ein Wasserstoffatom oder eine $C_1$-$C_6$-Alkylgruppe bedeutet, in Gegenwart von Pyridin unterwirft zur Bildung einer Verbindung der Formel (VII)

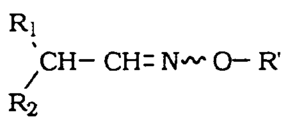 (VII)

in der $R_1$, $R_2$ und R' die oben angegebenen Bedeutungen besitzen, welche man durch katalytische Hydrierung oder in Gegenwart eines gemischten Alkalimetallhydrids reduziert zur Bildung eines Amins der Formel (VIII)

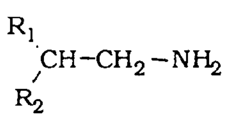 (VIII)

in der $R_1$ und $R_2$ die bezüglich der Formel (I) angegebenen Bedeutungen besitzen, welches man der Einwirkung einer Verbindung der Formel (IX)

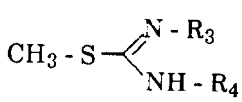 (IX)

in der $R_3$ und $R_4$ die bezüglich der Formel (I) angegebenen Bedeutungen besitzen, unterwirft, zur Bildung einer Verbindung der Formel (I/d), einem Sonderfall der Verbindungen der Formel (I)

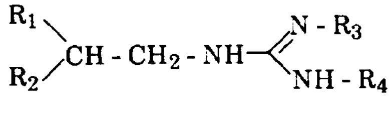 (I/d)

in der $R_1$, $R_2$, $R_3$ und $R_4$ die bezüglich der Formel (I) angegebenen Bedeutungen besitzen und A eine Gruppe der Formel $-CH_2-$ und R ein Wasserstoffatom bedeuten,
wobei die Verbindungen der Formeln (I/a), (I/c) und (I/d) gemeinsam die Verbindungen der Formel (I) darstellen, die man gegebenenfalls durch klassische Reinigungsverfahren reinigt, die man gewünschtenfalls mit Hilfe klassischer Trennverfahren in die Isomeren auftrennt und die man erforderlichenfalls in ihre Additionssalze mit einer pharmazeutisch annehmbaren Säure überführt.

**2.** Verfahren nach Anspruch 1 zur Herstellung von Verbindungen. worin $R_2$ eine Trifluormethylgruppe bedeutet. von deren Enantiomeren und Epimeren sowie von deren Additionssalzen mit einer pharmazeutisch annehmbaren Säure.

**3.** Verfahren nach Anspruch 1 zur Herstellung von Verbindungen, worin $R_1$ und $R_2$ jeweils eine Cyclopropylgruppe bedeuten, von deren Enantiomeren und Epimeren sowie von deren Additionssalzen mit einer pharmazeutisch annehmbaren Säure.

**4.** Verfahren nach Anspruch 1 zur Herstellung von Verbindungen, worin A eine Gruppe der Formel -CH=N- bedeutet, von deren Enantiomeren und Epimeren sowie von deren Additionssalzen mit einer pharmazeutisch annehmbaren Säure.

**5.** Verfahren nach Anspruch 1 zur Herstellung von Verbindungen, worin $R_3$ und $R_4$ jeweils ein Wasserstoffatom bedeuten, von deren Enantiomeren und Epimeren sowie von deren Additionssalzen mit einer pharmazeutisch annehmbaren Säure.

**6.** Verfahren nach Anspruch 1 zur Herstellung von Verbindungen. worin $R_3$ und $R_4$ gemeinsam mit den Stickstoffatomen, an die sie gebunden sind, einen Imidazolinring bilden, von deren Enantiomeren und Epimeren sowie von deren Additionssalzen mit einer pharmazeutisch annehmbaren Säure.

**7.** Verfahren nach einem der Ansprüche 1, 3, 4 oder 5 zur Herstellung von (2,2-Dicyclopropylethylidenyl)-aminoguanidin und dessen Additionssalzen mit einer pharmazeutisch annehmbaren Säure.

**Patentansprüche für folgenden Vertragsstaat : GR**

**1.** Verfahren zur Herstellung der Verbindungen der allgemeinen Formel (I):

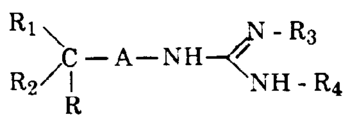

(I)

in der

| | |
|---|---|
| $R_1$ | - eine Phenylgruppe. die gegebenenfalls durch eines oder mehrere Halogenatome, geradkettige oder verzweigte $C_1$-$C_6$-Alkylgruppen, Hydroxylgruppen oder geradkettige oder verzweigte $C_1$-$C_6$-Alkoxygruppen substituiert ist, oder<br>- eine Cycloalkylgruppe mit 3 bis 7 Kohlenstoffatomen, |
| $R_2$ | eine Trifluormethylgruppe oder eine Cycloalkylgruppe mit 3 bis 4 Kohlenstoffatomen, |
| A | eine Gruppe der Formel $-CH_2-$, -C=N-, =N-, |
| $R_3$ und $R_4$, | die gleichartig oder verschieden sein können, Wasserstoffatome, geradkettige oder verzweigte $C_1$-$C_6$-Alkylgruppen oder gemeinsam mit den Stickstoffatomen, an die sie gebunden sind, einen Heterocyclus mit 5 oder 6 Kettengliedern und |
| R | entweder ein Wasserstoffatom dann, wenn A eine Gruppe der Formel $-CH_2-$ oder -CH=N- darstellt, oder eine Bindung der Gruppe A dann, wenn diese eine Gruppe der Formel =N- darstellt,<br>mit der Maßgabe bedeuten, daß, wenn A eine Gruppe der Formel =N- und $R_1$ eine gegebenenfalls substituierte Phenylgruppe darstellen, $R_2$ nicht gleichzeitig eine $C_3$-$C_4$-Cycloalkylgruppe bedeutet, |

deren Enantiomere und Epimere sowie ihrer Additionssalze mit einer pharmazeutisch annehmbaren Säure, **dadurch gekennzeichnet,** daß man als Ausgangsmaterial ein Keton der Formel (II):

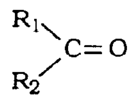 (II)

worin $R_1$ und $R_2$ die bezüglich der Formel (I) angegebenen Bedeutungen besitzen, verwendet. welches man

1/ **entweder**

der Einwirkung einer Verbindung der Formel (III)

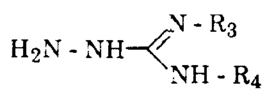 (III)

in der $R_3$ und $R_4$ die bezüglich der Formel (I) angegebenen Bedeutungen besitzen, in einem organischen Losungsmittel unterwirft zur Bildung einer Verbindung der Formel (I/a), einem Sonderfall der Verbindungen der Formel (I)

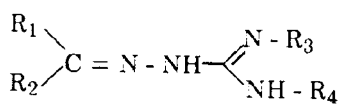 (I/a)

in der $R_1$, $R_2$, $R_3$ und $R_4$ die für die Formel (I) angegebenen Bedeutungen besitzen, A eine Gruppe der Formel =N- und R eine Bindung dieser Gruppe bedeuten,

2/ **oder**

der Einwirkung von Methoxymethyltriphenylphosphoniumchlorid unterwirft zur Bildung eines Enolethers der Formel (IV)

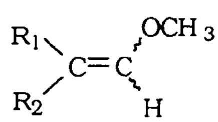 (IV)

in der $R_1$ und $R_2$ die bezüglich der Formel (I) angegebenen Bedeutungen besitzen,
welchen man in saurem Medium hydrolysiert zur Bildung eines Aldehyds der Formel (V)

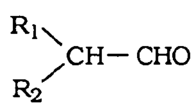 (V)

in der $R_1$ und $R_2$ die bezüglich der Formel (I) angegebenen Bedeutungen besitzen,
welchen man

entweder

der Einwirkung einer Verbindung der Formel (III)

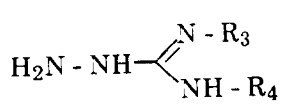 (III)

in der $R_3$ und $R_4$ die bezüglich der Formel (I) angegebenen Bedeutungen besitzen, unterwirft zur Bildung einer Verbindung der Formel (I/c), einem Sonderfall der Verbindungen der Formel (I)

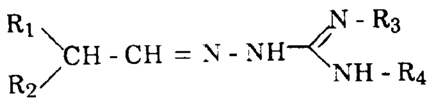

(I/c)

in der $R_1$, $R_2$, $R_3$ und $R_4$ die bezüglich der Formel (I) angegebenen Bedeutungen besitzen, und A eine Gruppe der Formel -CH=N- und R ein Wasserstoffatom bedeuten,

oder

der Einwirkung einer Verbindung der Formel (VI)

$$H_2N\text{-}O\text{-}R' \qquad (VI)$$

in der R' ein Wasserstoffatom oder eine $C_1$-$C_6$-Alkylgruppe bedeutet, in Gegenwart von Pyridin unterwirft zur Bildung einer Verbindung der Formel (VII)

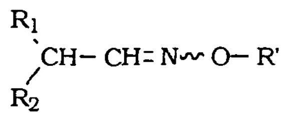

(VII)

in der $R_1$, $R_2$ und R' die oben angegebenen Bedeutungen besitzen. welche man durch katalytische Hydrierung oder in Gegenwart eines gemischten Alkalimetallhydrids reduziert zur Bildung eines Amins der Formel (VIII)

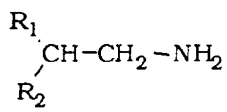

(VIII)

in der $R_1$ und $R_2$ die bezüglich der Formel (I) angegebenen Bedeutungen besitzen,
welches man der Einwirkung einer Verbindung der Formel (IX)

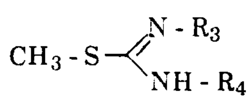

(IX)

in der $R_3$ und $R_4$ die bezüglich der Formel (I) angegebenen Bedeutungen besitzen, unterwirft, zur Bildung einer Verbindung der Formel (I/d), einem Sonderfall der Verbindungen der Formel (I)

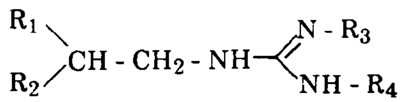

(I/d)

in der $R_1$, $R_2$, $R_3$ und $R_4$ die bezüglich der Formel (I) angegebenen Bedeutungen besitzen und A eine Gruppe der Formel -$CH_2$- und R ein Wasserstoffatom bedeuten,
wobei die Verbindungen der Formeln (I/a), (I/c) und (I/d) gemeinsam die Verbindungen der Formel (I) darstellen, die man gegebenenfalls durch klassische Reinigungsverfahren reinigt, die man gewünschtenfalls mit Hilfe klassischer Trennverfahren in die Isomeren auftrennt und die man erforderlichenfalls in ihre Additionssalze mit einer pharmazeutisch annehmbaren Säure überführt.

2. Verfahren nach Anspruch 1 zur Herstellung von Verbindungen. worin $R_2$ eine Trifluormethylgruppe bedeutet. von deren Enantiomeren und Epimeren sowie von deren Additionssalzen mit einer pharmazeutisch

annehmbaren Säure.

3. Verfahren nach Anspruch 1 zur Herstellung von Verbindungen, worin $R_1$ und $R_2$ jeweils eine Cyclopropylgruppe bedeuten, von deren Enantiomeren und Epimeren sowie von deren Additionssalzen mit einer pharmazeutisch annehmbaren Säure.

4. Verfahren nach Anspruch 1 zur Herstellung von Verbindungen, worin A eine Gruppe der Formel -CH=N- bedeutet, von deren Enantiomeren und Epimeren sowie von deren Additionssalzen mit einer pharmazeutisch annehmbaren Säure.

5. Verfahren nach Anspruch 1 zur Herstellung von Verbindungen. worin $R_3$ und $R_4$ jeweils ein Wasserstoffatom bedeuten. von deren Enantiomeren und Epimeren sowie von deren Additionssalzen mit einer pharmazeutisch annehmbaren Säure.

6. Verfahren nach Anspruch 1 zur Herstellung von Verbindungen, worin $R_3$ und $R_4$ gemeinsam mit den Stickstoffatomen, an die sie gebunden sind, einen Imidazolinring bilden, von deren Enantiomeren und Epimeren sowie von deren Additionssalzen mit einer pharmazeutisch annehmbaren Säure.

7. Verfahren nach einem der Ansprüche 1, 3, 4 oder 5 zur Herstellung von (2,2-Dicyclopropylethylidenyl)-aminoguanidin und dessen Additionssalzen mit einer pharmazeutisch annehmbaren Säure.

## Claims

### Claims for the following Contracting States : AT, BE, CH, DE, DK, FR, GB, IT, LI, LU, NL, SE

1. Compounds of the general formula (I):

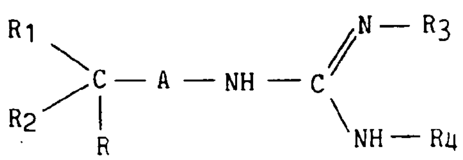

(I)

in which:

| | |
|---|---|
| $R_1$ | represents - a phenyl group optionally substituted by one or more halogen atoms, linear or branched ($C_1$-$C_6$)-alkyl groups, hydroxy groups, or linear or branched ($C_1$-$C_6$)-alkoxy groups; or<br>- a cycloalkyl group containing from 3 to 7 carbon atoms, |
| $R_2$ | represents a trifluoromethyl group or a cycloalkyl group containing 3 or 4 carbon atoms, |
| A | represents a group $-CH_2-$, -CH=N- or =N-, |
| $R_3$ and $R_4$, | which may be identical or different, represent a hydrogen atom or a linear or branched ($C_1$-$C_6$)-alkyl group or, together with the nitrogen atoms to which they are bonded, form a heterocycle having 5 or 6 ring members, |
| R | represents either a hydrogen atom when A represents a group $-CH_2-$ or -CH=N-, or a bond of the group A when the latter is represented by =N-,<br>with the proviso, however, that when A represents a group =N- and $R_1$ represents an optionally substituted phenyl group, $R_2$ does not at the same time represent a ($C_3$-$C_4$)-cycloalkyl group, |

their enantiomers and epimers and their addition salts with a pharmaceutically acceptable acid.

2. Compounds according to claim 1 in which $R_2$ represents a trifluoromethyl group, their enantiomers and epimers and their addition salts with a pharmaceutically acceptable acid.

3. Compounds according to claim 1 in which each of $R_1$ and $R_2$ represents a cyclopropyl group, their enantiomers and epimers and their addition salts with a pharmaceutically acceptable acid.

4. Compounds according to claim 1 in which A represents a group -CH=N-, their enantiomers and epimers and their addition salts with a pharmaceutically acceptable acid.

5. Compounds according to claim 1 in which each of $R_3$ and $R_4$ represents a hydrogen atom, their enantiomers and epimers and their addition salts with a pharmaceutically acceptable acid.

6. Compounds according to claim 1 in which $R_3$ and $R_4$, together with the nitrogen atoms to which they are bonded, form an imidazoline ring, their enantiomers and epimers and their addition salts with a pharmaceutically acceptable acid.

7. Compound according to any one of claims 1, 3, 4 and 5 which is (2,2-dicyclopropylethylidenyl)aminoguanidine, and its addition salts with a pharmaceutically acceptable acid.

8. Process for the preparation of the compounds of formula (I), characterised in that there is used as starting material a ketone of formula (II):

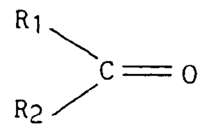

(II),

in which $R_1$ and $R_2$ have the same meaning as in formula (I),
which is subjected
1. <u>either</u>
to the action of a compound of formula (III) in an organic solvent

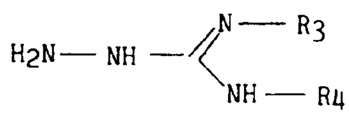

(III),

in which $R_3$ and $R_4$ have the same meaning as in formula (I),
to give a compound of formula (I/a), which is a particular case of the compounds of formula (I),

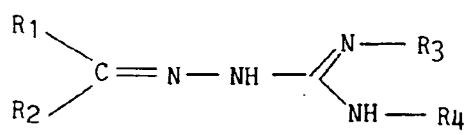

(I/a),

in which $R_1$, $R_2$, $R_3$ and $R_4$ have the same meaning as in formula (I), A represents a group =N- and R represents a bond of that group,
2. <u>or</u>
to the action of methoxymethyltriphenylphosphonium chloride to give the enol ether of formula (IV)

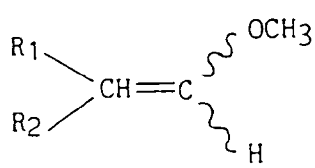

(IV),

in which $R_1$ and $R_2$ have the same meaning as in formula (I),
which is hydrolysed in an acid medium to give the aldehyde of formula (V)

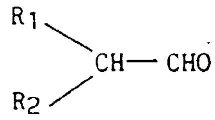

(V),

in which $R_1$ and $R_2$ have the same meaning as in formula (I), which is subjected

either

to the action of a compound of formula (III)

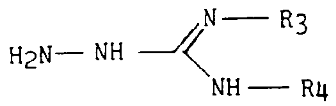

(III),

in which $R_3$ and $R_4$ have the same meaning as in formula (I), to give a compound of formula (I/c), which is a particular case of the compounds of formula (I),

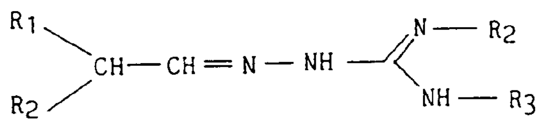

(I/c),

in which $R_1$, $R_2$, $R_3$ and $R_4$ have the same meaning as in formula (I), A represents a group -CH=N- and R represents a hydrogen atom,

or

to the action of a compound of formula (VI) in the presence of pyridine

$H_2N-O-R'$ (VI),

in which R' represents a hydrogen atom or a $(C_1-C_6)$-alkyl group, to give a compound of formula (VII)

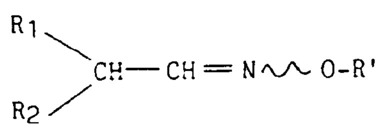

(VII),

in which $R_1$, $R_2$ and R' have the same meaning as above, which is reduced by catalytic hydrogenation or in the presence of an alkali metal mixed hydride to give an amine of formula (VIII)

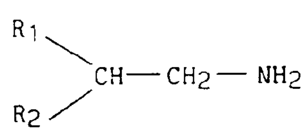

(VIII),

in which $R_1$ and $R_2$ have the same meaning as in formula (I), which is subjected to the action of a compound of formula (IX)

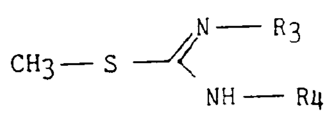

(IX),

in which $R_3$ and $R_4$ have the same meaning as in formula (I),

to give a compound of formula (I/d), which is a particular case of the compounds of formula (I),

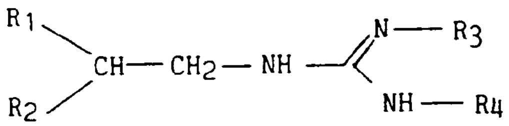

(I/d),

in which $R_1$, $R_2$, $R_3$ and $R_4$ have the same meaning as in formula (I), A represents a group $-CH_2-$ and R represents a hydrogen atom,
which compounds of formulae (I/a), (I/c) and (I/d) form the compounds of formula (I) in their entirety, which are purified, where applicable, by conventional purification methods, from which the isomers are separated, if desired, by conventional separation methods, and which are converted, if necessary, into their addition salts with a pharmaceutically acceptable acid.

9. Pharmaceutical compositions containing as active ingredient at least one compound according to any one of claims 1 to 7, on its own or in combination with one or more inert, non-toxic, pharmaceutically acceptable excipients or carriers.

10. Pharmaceutical compositions according to claim 9 for use in the treatment of depression, arterial hypertension, vascular disorders, diabetes, obesity and pulmonary diseases.

**Claims for the following Contracting State : ES**

1. Process for the preparation of the compounds of the general formula (I):

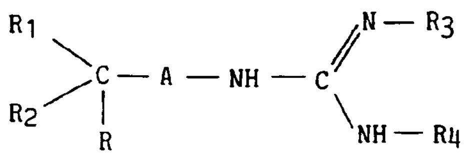

(I),

in which:

| | |
|---|---|
| $R_1$ | represents - a phenyl group optionally substituted by one or more halogen atoms, linear or branched $(C_1-C_6)$-alkyl groups, hydroxy groups, or linear or branched $(C_1-C_6)$-alkoxy groups; or<br>- a cycloalkyl group containing from 3 to 7 carbon atoms, |
| $R_2$ | represents a trifluoromethyl group or a cycloalkyl group containing 3 or 4 carbon atoms, |
| A | represents a group $-CH_2-$, $-CH=N-$ or $=N-$, |
| $R_3$ and $R_4$, | which may be identical or different, represent a hydrogen atom or a linear or branched $(C_1-C_6)$-alkyl group or, together with the nitrogen atoms to which they are bonded, form a heterocycle having 5 or 6 ring members, |
| R | represents either a hydrogen atom when A represents a group $-CH_2-$ or $-CH=N-$, or a bond of the group A when the latter is represented by $=N-$,<br>with the proviso, however, that when A represents a group $=N-$ and $R_1$ represents an optionally substituted phenyl group, $R_2$ does not at the same time represent a $(C_3-C_4)$-cycloalkyl group, |

their enantiomers and epimers and their addition salts with a pharmaceutically acceptable acid, characterised in that there is used as starting material a ketone of formula (II):

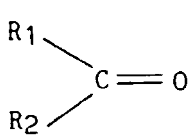

(II),

in which $R_1$ and $R_2$ have the same meaning as in formula (I),

which is subjected

1. <u>either</u>

to the action of a compound of formula (III) in an organic solvent

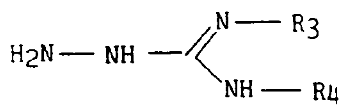

(III),

in which $R_3$ and $R_4$ have the same meaning as in formula (I),
to give a compound of formula (I/a), which is a particular case of the compounds of formula (I),

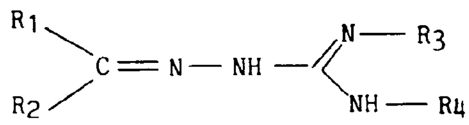

(I/a),

in which $R_1$, $R_2$, $R_3$ and $R_4$ have the same meaning as in formula (I), A represents a group =N- and R represents a bond of that group,

2. <u>or</u>

to the action of methoxymethyltriphenylphosphonium chloride to give the enol ether of formula (IV)

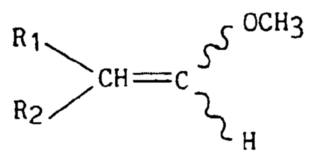

(IV),

in which $R_1$ and $R_2$ have the same meaning as in formula (I),
which is hydrolysed in an acid medium to give the aldehyde of formula (V)

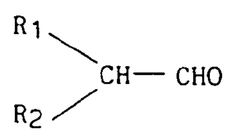

(V),

in which $R_1$ and $R_2$ have the same meaning as in formula (I),
which is subjected

<u>either</u>

to the action of a compound of formula (III)

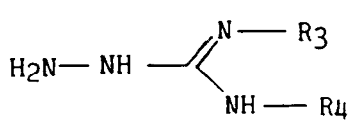

(III),

in which $R_3$ and $R_4$ have the same meaning as in formula (I),
to give a compound of formula (I/c), which is a particular case of the compounds of formula (I),

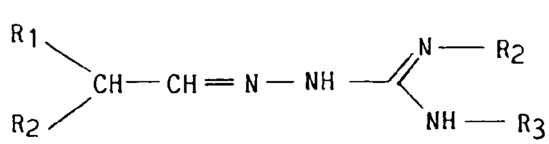

(I/c),

in which $R_1$, $R_2$, $R_3$ and $R_4$ have the same meaning as in formula (I), A represents a group -CH=N- and R represents a hydrogen atom,
<u>or</u>
to the action of a compound of formula (VI) in the presence of pyridine

$H_2N$-O-R' (VI),

in which R' represents a hydrogen atom or a ($C_1$-$C_6$)-alkyl group,
to give a compound of formula (VII)

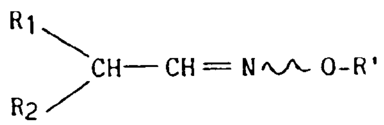

(VII),

in which $R_1$, $R_2$ and R' have the same meaning as above,
which is reduced by catalytic hydrogenation or in the presence of an alkali metal mixed hydride to give an amine of formula (VIII)

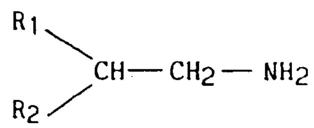

(VIII),

in which $R_1$ and $R_2$ have the same meaning as in formula (I),
which is subjected to the action of a compound of formula (IX)

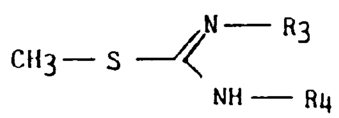

(IX),

in which $R_3$ and $R_4$ have the same meaning as in formula (I),
to give a compound of formula (I/d), which is a particular case of the compounds of formula (I),

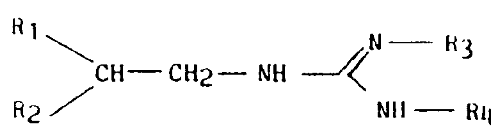

(I/d),

in which $R_1$, $R_2$, $R_3$ and $R_4$ have the same meaning as in formula (I), A represents a group -$CH_2$- and R represents a hydrogen atom,
which compounds of formulae (I/a), (I/c) and (I/d) form the compounds of formula (I) in their entirety, which are purified, where applicable, by conventional purification methods, from which the isomers are separated, if desired, by conventional separation methods, and which are converted, if necessary, into their addition salts with a pharmaceutically acceptable acid.

2. Process according to claim 1 for the preparation of compounds in which $R_2$ represents a trifluoromethyl group, their enantiomers and epimers and their addition salts with a pharmaceutically acceptable acid.

3. Process according to claim 1 for the preparation of compounds in which each of $R_1$ and $R_2$ represents a cyclopropyl group, their enantiomers and epimers and their addition salts with a pharmaceutically acceptable acid.

4. Process according to claim 1 for the preparation of compounds in which A represents a group -CH=N-,

their enantiomers and epimers and their addition salts with a pharmaceutically acceptable acid.

5. Process according to claim 1 for the preparation of compounds in which each of $R_3$ and $R_4$ represents a hydrogen atom, their enantiomers and epimers and their addition salts with a pharmaceutically acceptable acid.

6. Process according to claim 1 for the preparation of compounds in which $R_3$ and $R_4$, together with the nitrogen atoms to which they are bonded, form an imidazoline ring, their enantiomers and epimers and their addition salts with a pharmaceutically acceptable acid,

7. Process according to any one of claims 1, 3, 4 and 5 for the preparation of the compound which is (2,2-dicyclopropylethylidenyl)aminoguanidine, and its addition salts with a pharmaceutically acceptable acid.

**Claims for the following Contracting State : GR**

1. Process for the preparation of the compounds of the general formula (I):

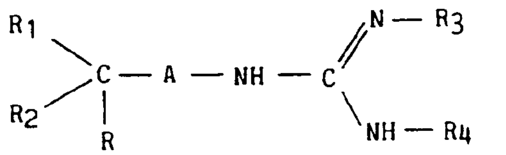

(I),

in which:

$R_1$ represents - a phenyl group optionally substituted by one or more halogen atoms, linear or branched ($C_1$-$C_6$)-alkyl groups, hydroxy groups, or linear or branched ($C_1$-$C_6$)-alkoxy groups; or
- a cycloalkyl group containing from 3 to 7 carbon atoms,

$R_2$ represents a trifluoromethyl group or a cycloalkyl group containing 3 or 4 carbon atoms,

A represents a group $-CH_2-$, $-CH{=}N-$ or $=N-$,

$R_3$ and $R_4$, which may be identical or different, represent a hydrogen atom or a linear or branched ($C_1$-$C_6$)-alkyl group or, together with the nitrogen atoms to which they are bonded, form a heterocycle having 5 or 6 ring members,

R represents either a hydrogen atom when A represents a group $-CH_2-$ or $-CH{=}N-$, or a bond of the group A when the latter is represented by $=N-$,
with the proviso, however, that when A represents a group $=N-$ and $R_1$ represents an optionally substituted phenyl group, $R_2$ does not at the same time represent a ($C_3$-$C_4$)-cycloalkyl group,

their enantiomers and epimers and their addition salts with a pharmaceutically acceptable acid, characterised in that there is used as starting material a ketone of formula (II):

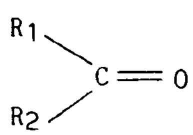

(II),

in which $R_1$ and $R_2$ have the same meaning as in formula (I), which is subjected

1. either

to the action of a compound of formula (III) in an organic solvent

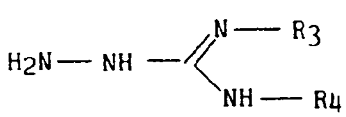

(III),

in which $R_3$ and $R_4$ have the same meaning as in formula (I),
to give a compound of formula (I/a), which is a particular case of the compounds of formula (I),

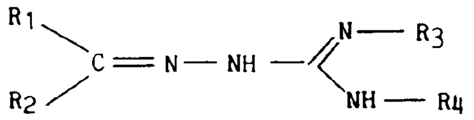

(I/a),

in which $R_1$, $R_2$, $R_3$ and $R_4$ have the same meaning as in formula (I), A represents a group =N- and R represents a bond of that group,
2. or
to the action of methoxymethyltriphenylphosphonium chloride to give the enol ether of formula (IV)

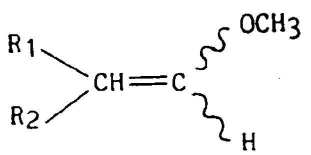

(IV),

in which $R_1$ and $R_2$ have the same meaning as in formula (I),
which is hydrolysed in an acid medium to give the aldehyde of formula (V)

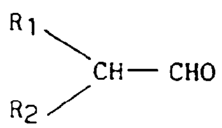

(V),

in which $R_1$ and $R_2$ have the same meaning as in formula (I),
which is subjected
either
to the action of a compound of formula (III)

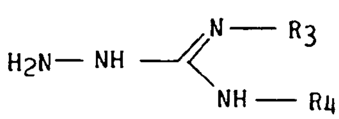

(III),

in which $R_3$ and $R_4$ have the same meaning as in formula (I),
to give a compound of formula (I/c), which is a particular case of the compounds of formula (I),

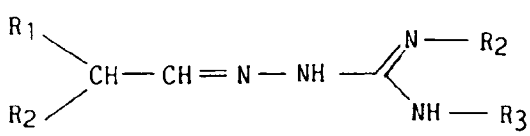

(I/c),

in which $R_1$, $R_2$, $R_3$ and $R_4$ have the same meaning as in formula (I), A represents a group -CH=N- and R represents a hydrogen atom,
or
to the action of a compound of formula (VI) in the presence of pyridine

$H_2N\text{-}O\text{-}R'$ (VI),

in which R' represents a hydrogen atom or a $(C_1\text{-}C_6)$-alkyl group,
to give a compound of formula (VII)

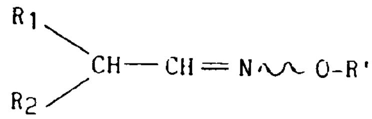

(VII),

in which $R_1$, $R_2$ and R' have the same meaning as above, which is reduced by catalytic hydrogenation or in the presence of an alkali metal mixed hydride to give an amine of formula (VIII)

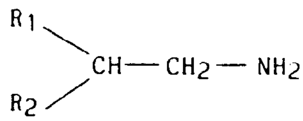

(VIII),

in which $R_1$ and $R_2$ have the same meaning as in formula (I), which is subjected to the action of a compound of formula (IX)

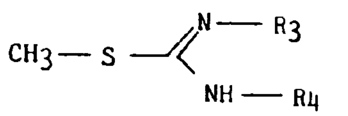

(IX),

in which $R_3$ and $R_4$ have the same meaning as in formula (I), to give a compound of formula (I/d), which is a particular case of the compounds of formula (I),

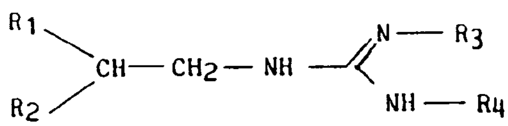

(I/d),

in which $R_1$, $R_2$, $R_3$ and $R_4$ have the same meaning as in formula (I), A represents a group $-CH_2-$ and R represents a hydrogen atom, which compounds of formulae (I/a), (I/c) and (I/d) form the compounds of formula (I) in their entirety, which are purified, where applicable, by conventional purification methods, from which the isomers are separated, if desired, by conventional separation methods, and which are converted, if necessary, into their addition salts with a pharmaceutically acceptable acid.

2. Process according to claim 1 for the preparation of compounds in which $R_2$ represents a trifluoromethyl group, their enantiomers and epimers and their addition salts with a pharmaceutically acceptable acid.

3. Process according to claim 1 for the preparation of compounds in which each of $R_1$ and $R_2$ represents a cyclopropyl group, their enantiomers and epimers and their addition salts with a pharmaceutically acceptable acid.

4. Process according to claim 1 for the preparation of compounds in which A represents a group -CH=N-, their enantiomers and epimers and their addition salts with a pharmaceutically acceptable acid.

5. Process according to claim 1 for the preparation of compounds in which each of $R_3$ and $R_4$ represents a hydrogen atom, their enantiomers and epimers and their addition salts with a pharmaceutically acceptable acid.

6. Process according to claim 1 for the preparation of compounds in which $R_3$ and $R_4$, together with the nitrogen atoms to which they are bonded, form an imidazoline ring, their enantiomers and epimers and their addition salts with a pharmaceutically acceptable acid.

7. Process according to any one of claims 1, 3, 4 and 5 for the preparation of the compound which is (2,2-dicyclopropylethylidenyl)aminoguanidine, and its addition salts with a pharmaceutically acceptable acid.